(19) Europäisches Patentamt
European Patent Office
Office européen des brevets

(11) **EP 4 285 824 B1**

(12) **EUROPÄISCHE PATENTSCHRIFT**

(45) Veröffentlichungstag und Bekanntmachung des Hinweises auf die Patenterteilung:
**20.08.2025 Patentblatt 2025/34**

(21) Anmeldenummer: **22176539.9**

(22) Anmeldetag: **31.05.2022**

(51) Internationale Patentklassifikation (IPC):
***A61B 6/00*** *(2024.01)* ***A61B 6/03*** *(2006.01)*

(52) Gemeinsame Patentklassifikation (CPC):
**A61B 6/56; A61B 6/035; A61B 6/54**

(54) **GANTRYANTRIEB MIT INTEGRIERTER LEISTUNGSÜBERTRAGUNG**

GANTRY DRIVE WITH INTEGRATED POWER TRANSMISSION

ENTRAÎNEMENT DE PORTIQUE À TRANSMISSION INTÉGRÉE DE PUISSANCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.12.2023 Patentblatt 2023/49**

(73) Patentinhaber: **Siemens Healthineers AG**
**91301 Forchheim (DE)**

(72) Erfinder: **Weidinger, Thomas**
**91052 Erlangen (DE)**

(74) Vertreter: **Siemens Healthineers**
**Patent Attorneys**
**Postfach 22 16 34**
**80506 München (DE)**

(56) Entgegenhaltungen:
**EP-A2- 0 181 176     DE-A1- 102014 201 805**
**JP-A- 2004 202 092     US-A1- 2002 031 201**
**US-A1- 2017 214 279**

**Beschreibung**

Die Erfindung betrifft einen Gantryantrieb, eine Computertomographieeinrichtung, ein Verfahren zum Erzeugen einer Drehbewegung eines Drehstromrotors mittels eines Gantryantriebs und ein zugehöriges Computerprogrammprodukt.

Eine herkömmliche Gantry für eine Computertomographieeinrichtung weist typischerweise zwei Trägerringe auf, welche um einen tunnelförmigen Hohlraum herum angeordnet sind. Die beiden Trägerringe sind üblicherweise relativ zueinander drehbar angeordnet, so dass einer der beiden Trägerringe einen stationären Trägerring und der andere der beiden Trägerringe einen rotierenden Trägerring bildet.

Wenn am rotierenden Trägerring ein Röntgenstrahler und ein Röntgendetektor angeordnet sind, wird üblicherweise der rotierende Trägerring um den Hohlraum herum mit typischerweise mindestens einer halben Umdrehung, insbesondere mit einer Vielzahl an Umdrehungen in einer Drehbewegung rotiert. Zur Durchführung der Drehbewegung ist eine mechanische Antriebsleistung vonnöten.

Vom Röntgenstrahler erzeugte Röntgenstrahlen durchleuchten bei der Drehbewegung beispielsweise einen im Hohlraum angeordneten Patienten (oder ein derart angeordnetes Objekt) und von dem mit den Röntgenstrahlen durchleuchteten Patienten abhängige Schwächungsprofile können mittels des Röntgendetektors erfasst und für eine Rekonstruktion eines tomographischen Bildes verwendet werden. Ein solcher herkömmlicher Röntgenstrahler oder Röntgendetektor ist eine Nutzlast, welche auf dem rotierenden Trägerring mit elektrischer Leistung im Betrieb versorgt wird.

EP 0 181 176 A2 offenbart einen Computertomographie-Scanner, welcher einen Rahmen umfasst, der drehbar auf einer Gantry montiert ist und eine Röntgenquelle trägt, die so angeordnet ist, dass sie Strahlung durch ein Subjekt auf ein Detektorarray aus verschiedenen Richtungen leitet, während der Rahmen rotiert, wobei ein Elektromotor zum Drehen des Rahmens relativ zur Gantry vorgesehen ist, wobei eines seiner felderzeugenden und feldempfindlichen Teile direkt auf einem Teil eines Lagers montiert ist, über das der Rahmen auf der Gantry montiert ist.

Aus US 2002 031 201 A1 ist bekannt, als Rotor das Drehelement zu verwenden, das die Röntgenstrahlung und die Röntgendetektoreinheit trägt und um ein Objekt rotiert. Der Rotor ist mit einem Rotorkern und mehreren mit dem Kern verbundenen Leitern ausgestattet. Der Stator weist mindestens einen Satz aus Statorkern und Statorwicklung auf, wobei der mindestens eine Satz aus Statorkern und Statorwicklung zum Festklemmen des Rotors geeignet und an gegenüberliegenden Positionen angeordnet ist. Der Statorwicklung wird ein dreiphasiger Wechselstrom zugeführt, um ein rotierendes Magnetfeld zu erzeugen, um den Rotor zu drehen und dadurch das Drehelement mit hoher Geschwindigkeit zu drehen. Da die Scanzeit verkürzt werden kann, kann der Röntgen-CT-Scanner bewegliche Organe wie das Herz scannen.

In JP 2004 202 092 A ist ein Spannungsdetektor bekannt, welcher die Spannung einer kommerziellen Stromversorgung an einem Eingangsanschluss eines Wandlers erkennt. Wenn eine erfasste Versorgungsspannung VAC unter einer Spannung Vt liegt, bestimmt ein Abschnitt zur Bestimmung des Steigungswinkels einen Steigungswinkel zur Durchführung der Steigungsphasensteuerung eines bürstenlosen Elektromotors, der den mit einer Röntgenröhre ausgestatteten Drehrahmen antreibt. Ein Phasenbestimmungsabschnitt bestimmt die Phase der Anschlussspannungen Vu, Vv und Vw des bürstenlosen Elektromotors, sodass die aktuelle Phase in Bezug auf einen Drehwinkel gleich dem Steigungswinkel ist.

DE 10 2014 201 805 A1 gibt ein bildgebendes medizinisches Gerät an, welches einen feststehenden Geräteteil und einen hierzu beweglich gelagerten beweglichen Geräteteil umfasst, wobei am feststehenden Geräteteil eine Leistungsquelle zur Bereitstellung von Energie angeordnet ist, wobei am beweglichen Geräteteil eine Anzahl von elektrischen Hauptverbrauchern, eine Anzahl von elektrischen Nebenverbrauchern und ein Energiespeicher angeordnet sind, wobei zwischen dem feststehenden Geräteteil und dem beweglichen Geräteteil eine Energieübertragungsstrecke angeordnet ist, die dazu eingerichtet ist, Energie vom feststehenden Geräteteil auf den beweglichen Geräteteil zu übertragen, wobei eine Energie abgebende Komponente der Energieübertragungsstrecke am feststehenden Geräteteil mit der Leistungsquelle verbunden ist, wobei eine Energie aufnehmende Komponente der Energieübertragungsstrecke am beweglichen Geräteteil mit dem oder jedem Hauptverbraucher und mit dem oder jedem Nebenverbraucher über Schaltmittel schaltbar verbunden und mit dem Energiespeicher verbunden ist, und wobei der Energiespeicher mit dem oder jedem Nebenverbraucher schaltbar verbunden ist.

US 2017 214 279 A1 umfasst in einer Implementierung einen Direktantriebsmotor zur Verwendung in einer CT-Gantry und verfügt über eine segmentierte Motorstatorbaugruppe. Die Statorsegmente sind in Reihe geschaltet. Die Statorsegmente können nicht unabhängig voneinander betrieben werden, sondern sind stattdessen in Reihe mit einem einzigen Frequenzregler verbunden und werden von diesem betrieben.

Eine herkömmliche Gantry weist daher typischerweise zwei voneinander separate Leistungsübertragungssysteme auf, insbesondere ein System für das Bereitstellen der mechanischen Antriebsleistung und ein weiteres System für das Übertragen der elektrischen Versorgungsleistung. Grundsätzlich ist eine Vielzahl an Varianten solcher herkömmlicher Gantryantriebe zum Übertragen der mechanischen Antriebsleistung, welche zur Drehbewegung des rotierenden Trägerrings verwendet wird, bekannt. Der herkömmliche Gantryantrieb kann insbesondere ein Direktantrieb sein. Der herkömmliche Direktantrieb kann beispielsweise als permanenterregter Synchronmotor ausgeführt sein, wobei Magneten auf dem rotierenden Teil angeordnet sind und somit eine Leistung für den Direktantrieb lediglich auf dem stationären Teil bereitgestellt wird. Alternativ kann die herkömmliche Gantry über ein Getriebe und/oder einen Riemen angetrieben sein. Beispielsweise sind genannt Zahnradkupplungen, Keilriemenantriebe, etc.

Das Übertragen der elektrischen Versorgungsleistung kann grundsätzlich kontaktlos oder kontaktbehaftet erfolgen. Kontaktbehaftete Übertrager umfassen typischerweise eine Anordnung einer Bürste und eines Schleifrings, welche zueinander im Kontakt stehen und verdrehbar sind und dabei die elektrische Versorgungsleistung in Gleich- oder Wechselspannung zwischen dem stationären Trägerring und dem rotierenden Trägerring übertragen können. Eine kontaktlose Leistungsübertragung erfolgt regelmäßig mittels in Magnetkernen eingelegten Wicklungen, welche mit Wechselspannung betrieben werden, und jeweils gegenüber voneinander am stationären Trägerring und am rotierenden Trägerring angeordnet sind. Diesbezüglich sind dem Fachmann ebenfalls verschiedene Varianten aus dem Stand der Technik bekannt.

Zusätzlich kann eine herkömmliche Gantry ein Datenübertragungssystem zum Übertragen von Daten, insbesondere der erfassten Schwächungsprofile, umfassen. Ein solches Datenübertragungssystem kann einen kapazitiven, resistiven, induktiven oder optischen Sender bzw. Empfänger umfassen.

Der herkömmliche Gantryantrieb ist typischerweise dazu ausgelegt, in einer Beschleunigungsphase eine Drehbewegung des rotierenden Trägerrings erzeugen zu können. Insbesondere bei der Beschleunigung aus dem Stillstand heraus ist eine im Vergleich zu einem späteren Zeitpunkt, in welchem die Sollgeschwindigkeit des rotierenden Trägerrings erreicht ist, hohe mechanische Antriebsleistung nötig. Entsprechend wird in einem herkömmlichen Gantryantrieb eine Leistungsreserve vorgehalten, welche insbesondere in der Beschleunigungsphase abgerufen wird, aber in einer an die Beschleunigungsphase anschließenden Fortlaufphase zu einem geringen Grad benötigt wird.

Gleichzeitig benötigt das System für das Übertragen der elektrischen Versorgungsleistung einen signifikanten Anteil an Bauraum. Je nach Konfiguration des Übertragers fällt ein entsprechender Wartungsaufwand oder Verschleiß an, beispielsweise verursacht durch die Reibung zwischen Bürste und Schleifring.

Der Erfindung liegt die Aufgabe zu Grunde, einen Gantryantrieb, eine Computertomographieeinrichtung, ein Verfahren zum Erzeugen einer Drehbewegung eines Drehstromrotors mittels eines Gantryantriebs und ein zugehöriges Computerprogrammprodukt anzugeben, bei welchen weniger Bauraum benötigt wird.

Die Aufgabe wird durch die Merkmale der unabhängigen Ansprüche gelöst. Vorteilhafte Ausgestaltungen sind in den Unteransprüchen beschrieben.

Der erfindungsgemäße Gantryantrieb weist

- einen Drehstromstator,
- einen Drehstromrotor,
- einen tunnelförmigen Hohlraum zur Aufnahme eines Patienten und
- eine Regeleinheit auf,
- wobei der Drehstromrotor den Hohlraum räumlich umgibt,
- wobei der Drehstromstator und der Drehstromrotor einen Drehstrommotor zum Bereitstellen einer Drehfeldleistung bilden,
- wobei mittels der Drehfeldleistung einerseits, in Form eines ersten Anteils, eine mechanische Antriebsleistung zur Erzeugung einer Drehbewegung des Drehstromrotors und andererseits, in Form eines zweiten Anteils, eine elektrische Versorgungsleistung einer Nutzlasteinrichtung bereitstellbar ist,
- wobei die Regeleinheit dazu ausgebildet ist, den ersten Anteil und den zweiten Anteil zu variieren.

Das erfindungsgemäße Verfahren zum Erzeugen der Drehbewegung des Drehstromrotors des Gantryantriebs umfasst folgende Schritte:

- Bereitstellen der Drehfeldleistung in dem Drehstrommotor derart, dass einerseits ein erster Anteil der Drehfeldleistung als mechanische Antriebsleistung zur Erzeugung der Drehbewegung des Drehstromrotors des Drehstrommotors wirkt und dass andererseits ein zweiter Anteil der Drehfeldleistung als elektrische Versorgungsleistung der Nutzlasteinrichtung wirkt,
- Variieren des ersten Anteils und des zweiten Anteils.

In anderen Worten wird die Drehfeldleistung in dem Drehstrommotor insbesondere derart bereitgestellt, dass mittels der Drehfeldleistung einerseits, in Form des ersten Anteils, die mechanische Antriebsleistung zur Erzeugung der Drehbewegung des Drehstromrotors des Drehstrommotors und andererseits, in Form des zweiten Anteils, die elektrische Versorgungsleistung der Nutzlasteinrichtung bereitgestellt wird. Mittels der bereitgestellten Drehfeldleistung kann insbesondere der Drehstrommotor mit der mechanischen Antriebsleistung angetrieben und die Nutzlasteinrichtung mit der elektrischen Versorgungsleistung betrieben werden.

Das erfindungsgemäße Computerprogrammprodukt, welches direkt in einen Speicher der Recheneinheit der Regeleinheit des Gantryantriebs ladbar ist, weist Programmcodemittel auf, um das Verfahren auszuführen, wenn das Computerprogrammprodukt in der Recheneinheit ausgeführt wird.

Der Gantryantrieb bzw. die Verwendung eines solchen Gantryantriebs bietet insbesondere den Vorteil, dass keine separaten Systeme für eine mechanische sowie elektrische Leistungsübertragung vorzusehen sind. In anderen Worten entfällt insbesondere der kontaktlose oder kontaktbehaftete separate Leistungsübertrager, weil die elektrische Versorgungsleistung mittels des Gantryantriebs bereitstellbar ist. In den Gantryantrieb ist insbesondere ein kontaktloser Leistungsübertrager integriert. In anderen Worten wirkt der Gantryantrieb zusätzlich als kontaktloser Leistungsübertrager. Dadurch wird typischerweise Bauraum eingespart.

Vorzugsweise werden insbesondere beim erfindungsgemäßen Gantryantrieb für die Beschleunigungsphase vorgehaltene Leistungsreserven auch nach der Beschleunigungsphase besser ausgenutzt. Insbesondere kann die Gesamtleistung des Direktantriebs erhöht werden aufgrund der Bauraumersparnis, was vorteilhafterweise zu einer schnelleren Beschleunigung des Drehstromrotors und/oder zu einem höheren Maximalwert der elektrischen Versorgungsleistung führt. Eine Beschleunigungszeit im Vergleich zu einem herkömmlichen Gantryantrieb kann vorteilhafterweise um den Faktor 2, besonders vorteilhafterweise um den Faktor 4 bis 6 verringert werden. Die Beschleunigungszeit des erfindungsgemäßen Gantryantriebs beträgt insbesondere weniger als 30s, vorzugsweise weniger als 10s, besonders vorteilhafterweise weniger als 5s. Vorzugsweise verringert sich oder entfällt der Wartungsaufwand ohne Bürste und Schleifring.

Der Gantryantrieb ist insbesondere ein Antrieb zum Antreiben einer Gantry. Der Gantryantrieb ist insbesondere ein Direktantrieb. Der Drehstromrotor wird insbesondere direkt vom Drehstromstator angetrieben. Die Gantry ist insbesondere für eine Computertomographieeinrichtung eingerichtet. Der Gantryantrieb weist eine derartige mechanische Antriebsleistung auf, dass die Drehbewegung um 360°, was einer Rotationsperiode entspricht, in weniger als 2 s, typischerweise weniger als 1 s, vorzugsweise weniger als 0,5 s erfolgt. In anderen Worten ist die mechanische Antriebsleistung so hoch, dass die Rotationsperiode sich 0,2 s annähern kann. Die mechanische Antriebsleistung kann vorzugsweise so hoch sein, dass eine g-Kraft an der Peripherie im Betrieb der Gantry 10 g, 20 g und/oder 50 g übersteigt.

Der tunnelförmige Hohlraum ist üblicherweise eine zentrale Ausnehmung im Gantryantrieb. Ein Durchmesser des Hohlraums ist typischerweise größer 10 cm und/oder oder kleiner 150 cm. Eine Drehachse des Drehstromrotors entspricht typischerweise einer Mittenachse des tunnelförmigen Hohlraums. Grundsätzlich ist es denkbar, dass zusätzlich der Drehstromstator den Hohlraum räumlich umgibt. In dem tunnelförmigen Hohlraum ist insbesondere der Patient lagerbar. Anstatt eines Patienten kann alternativ ein Untersuchungsobjekt, beispielsweise für eine Werkstoffprüfung, in dem tunnelförmigen Hohlraum angeordnet sein.

Der Drehstromstator und der Drehstromrotor bilden den Drehstrommmotor, also eine elektrische Maschine. Der Drehstrommotor kann zusätzlich zum Drehstromstator und zum Drehstromrotor zumindest eine weitere Komponente aufweisen, welche gemeinsam mit dem Drehstromstator und dem Drehstromrotor zum Bereitstellen der Drehfeldleistung ausgebildet ist. Üblicherweise besteht der Drehstrommotor nicht ausschließlich aus dem Drehstromstator und dem Drehstromrotor. In anderen Worten sind der Drehstromstator und der Drehstromrotor wesentliche, aber nicht die einzigen Komponenten eines Drehstrommmotors.

Das Bereitstellen der Drehfeldleistung umfasst insbesondere ein Erzeugen der Drehfeldleistung und/oder ein Aufnehmen der Drehfeldleistung. Das Bereitstellen der Drehfeldleistung umfasst insbesondere ein Erzeugen eines Drehfelds, welches typischerweise dazu geeignet ist, an einer die Drehfeldleistung aufnehmenden Einheit Arbeit zu verrichten. Das Bereitstellen der Drehfeldleistung ermöglicht vorzugsweise das Übertragen der mechanischen Antriebsleistung und der elektrischen Versorgungsleistung.

Der Drehstrommotor ist insbesondere symmetrisch aufgebaut. Das bedeutet, dass die Drehfeldleistung insbesondere zwischen dem Drehstromstator und dem Drehstromrotor hin oder her übertragen werden kann. In anderen Worten kann der Drehstromstator zur Erzeugung der Drehfeldleistung und der Drehstromrotor zur Aufnahme der Drehfeldleistung ausgebildet sein. Alternativ oder zusätzlich kann der Drehstromrotor zur Erzeugung der Drehfeldleistung und der Drehstromstator zur Aufnahme der Drehfeldleistung ausgebildet sein. Je nach Konfiguration des Gantryantriebs kann die Drehfeldleistung durch den Drehstromstator und den Drehstromrotor bereitstellbar sein.

Statorseitig ist die Eingangsleistung für den Drehstrommotor beispielsweise von einem Netzanschluss und/oder einem Energiepuffer bereitstellbar. Der Normalbetrieb des Gantryantriebs ist insbesondere der Netzbetrieb, wenn die Eingangsleistung von dem Netzanschluss bereitgestellt wird. Rotorseitig ist die Eingangsleistung für den Drehstrommotor typischerweise von einem Energiepuffer bereitstellbar. Ein solcher Betrieb des Gantryantriebs kann insbesondere USV-Betrieb genannt sein. Der Betrieb des Drehstrommotors, insbesondere des Drehstromstators und/oder des Drehstromrotors, ist typischerweise verlustbehaftet. Der Betrag der erzeugten Drehfeldleistung ist typischerweise geringer als der Betrag der Eingangsleistung.

Der Drehstrommotor weist insbesondere das Antriebsprinzip einer Asynchronmaschine auf und/oder ist eine Asynchronmaschine. Der Drehstrommotor ist insbesondere ein Asynchronmotor. Die Drehstromwicklungen bilden insbesondere dreisträngige Motorwicklungen. Der Drehstrommotor ist üblicherweise mit drei Phasen aufgebaut. Grundsätzlich ist eine andere Phasenanzahl, beispielsweise zwei Phasen oder mehr als drei Phasen, insbesondere sechs Phasen, möglich. Die in der vorliegenden Anmeldung verwendeten Begriffe, insbesondere diejenigen welche das Präfix "Dreh" umfassen, beispielsweise die Begriffe Drehstrommotor, Drehstromstator, Drehstromrotor, Drehfeldleistung, Drehstromwicklung, umfassen typischerweise einerseits den jeweiligen Betrieb mit drei Phasen und andererseits den jeweiligen Betrieb mit einer Phasenanzahl ungleich drei.

Der Drehstrommotor, insbesondere die Drehstromwicklungen, können trommelförmig oder scheibenförmig ausgerichtet sein.

Unter der trommelförmigen Ausrichtung wird bekannterweise verstanden, dass die Drehfeldleistung im Wesentlichen in radialer Richtung übertragen wird, während bei der scheibenförmigen Ausrichtung die Drehfeldleistung im Wesentlichen in axialer Richtung übertragen wird.

Durch das Bereitstellen der mechanischen Antriebsleistung wird insbesondere der Drehstromrotor relativ zum Drehstromstator angetrieben. Je nach Wahl des Bezugssystems kann der Drehstromrotor bezogen auf den Drehstromstator sich bewegen oder umgekehrt. Der Drehstromrotor ist insbesondere durch die bereitgestellte mechanische Antriebsleistung antreibbar, wobei der Drehstromrotor typischerweise eine Drehbewegung ausführt. Die Drehbewegung ist insbesondere eine Relativbewegung. Durch das Bereitstellen der elektrischen Versorgungsleistung wird insbesondere die Nutzlasteinrichtung betrieben. Die Nutzlasteinrichtung ist insbesondere durch die bereitgestellte elektrische Versorgungsleistung betreibbar oder im Betrieb.

Der Drehstromstator ist insbesondere hochpolig und/oder weist zumindest eine Drehstromwicklung auf, welche typischerweise in oder um ein Statorjoch herum angeordnet ist. Das Statorjoch weist beispielsweise Eisen auf. Vorzugsweise weist der Drehstromstator zumindest je eine Drehstromwicklung für die drei Phasen eines Netzanschlusses auf, wobei der Anschluss des Drehstromstators an den Netzanschluss üblicherweise mittels eines stationären Umrichters erfolgt. Der Drehstromstator verwendet typischerweise eine insbesondere elektrische Eingangsleistung für die Erzeugung der Drehfeldleistung, insbesondere im Netzbetrieb.

Der Drehstromrotor nimmt im Betrieb insbesondere die Drehfeldleistung auf. Die Aufnahme der Drehfeldleistung ermöglicht insbesondere das Bereitstellen der mechanischen Antriebsleistung und der elektrischen Versorgungsleistung. Die Aufnahme der Drehfeldleistung erfolgt typischerweise über einen Luftspalt zwischen dem Drehstromstator oder dem Drehstromrotor hinweg. Die Aufnahme der Drehfeldleistung erfolgt insbesondere kontaktlos. Die Verfügbarkeit der mechanischen Antriebsleistung und/oder der elektrischen Versorgungsleistung hängt insbesondere von der Verfügbarkeit der Drehfeldleistung ab.

Der Drehstromrotor weist typischerweise dieselbe Polzahl auf wie der Drehstromstator und/oder weist zumindest eine Drehstromwicklung auf, welche typischerweise in oder um ein Rotorjoch herum angeordnet ist. Das Rotorjoch weist beispielsweise Eisen auf. Vorzugsweise weist der Drehstromrotor zumindest je eine Drehstromwicklung für die drei Phasen des Netzanschlusses auf.

Der Gantryantrieb ist insbesondere dazu eingerichtet, mittels der Drehfeldleistung einerseits, in Form des ersten Anteils, die mechanische Antriebsleistung zur Erzeugung der Drehbewegung des Drehstromrotors und andererseits, in Form des zweiten Anteils, die elektrische Versorgungsleistung der Nutzlasteinrichtung bereitzustellen. Das Bereitstellen der mechanischen Antriebsleistung einerseits und der elektrischen Versorgungsleistung andererseits bedeutet insbesondere, dass die vom Drehstrommotor bereitstellbare oder bereitgestellte, vorzugsweise die vom Drehstromstator erzeugbare oder erzeugte und/oder vom Drehstromrotor aufnehmbare oder aufgenommene, Drehfeldleistung zumindest teilweise in mechanische Antriebsleistung und zumindest teilweise in elektrische Versorgungsleistung umwandelbar ist. Die bereitgestellte Drehfeldleistung lässt sich insbesondere in den ersten Anteil der Drehfeldleistung und in den zweiten Anteil der Drehfeldleistung unterteilen. Die bereitgestellte Drehfeldleistung wirkt insbesondere als mechanische Antriebsleistung und insbesondere gleichzeitig als elektrische Versorgungsleistung. Der erste Anteil entspricht insbesondere der mechanischen Antriebsleistung und der zweite Anteil entspricht insbesondere der elektrischen Versorgungsleistung. Die am Drehstromstator aufnehmbare oder aufgenommene Eingangsleistung wird typischerweise in der Drehfeldleistung bereitgestellt, welche am Drehstromrotor teils-teils in mechanischer Arbeitsleistung und in elektrischer Versorgungsleistung bereitgestellt oder bereitstellbar sein kann. Grundsätzlich ist ein derartiges Bereitstellen an Drehfeldleistung, mechanischer Antriebsleistung oder elektrischer Versorgungsleistung verlustbehaftet.

Mittels der mechanischen Antriebsleistung ist insbesondere die Drehbewegung des Drehstromrotors relativ zum Drehstromstator erzeugbar. Das Erzeugen der Drehbewegung erfolgt insbesondere im Motorbetrieb des Gantryantriebs und umfasst insbesondere ein Erhöhen der Drehgeschwindigkeit in der Beschleunigungsphase und/oder ein Aufrechterhalten der Drehgeschwindigkeit nach der Beschleunigungsphase in der Fortlaufphase. Das Erhöhen der Drehgeschwindigkeit erfordert typischerweise mehr mechanische Antriebsleistung als das Aufrechterhalten der Drehgeschwindigkeit. Das Aufrechterhalten der Drehgeschwindigkeit in der Fortlaufphase kann einen Zeitraum, in welchem die mechanische Antriebsleistung gleich null ist, und einen anderen Zeitraum, in welchem die mechanische Antriebsleistung größer null ist, umfassen. Grundsätzlich ist es denkbar, dass mittels der mechanischen Antriebsleistung die Drehgeschwindigkeit verringert und/oder die Drehbewegung gänzlich unterbunden wird, was beispielsweise im Generatorbetrieb des Gantryantriebs erfolgt.

Ein Zeitraum nach der Beschleunigungsphase kann insbesondere für einen Volllastbetrieb der Nutzlasteinrichtung vorgesehen sein. Der Zeitraum nach der Beschleunigungsphase, insbesondere die Fortlaufphase, kann insbesondere als Volllastbetriebsphase bezeichnet werden. Der Volllastbetrieb der Nutzlasteinrichtung ist insbesondere derart definiert, dass die Nutzlasteinrichtung beim Volllastbetrieb in der Fortlaufphase mehr elektrische Versorgungsleistung verbraucht als in der Beschleunigungsphase. In der Volllastbetriebsphase kann die elektrische Versorgungsleistung beispielsweise einen Maximalwert erreichen. Nach einer Fortlaufphase bzw. Volllastbetriebsphase kann erneut eine Beschleunigungsphase stattfinden. Beschleunigungsphasen und Fortlaufphasen bzw. Volllastbetriebsphasen können sich insbesondere regelmäßig abwechseln und/oder getaktet auftreten.

In der Beschleunigungsphase ist die elektrische Versorgungsleistung typischerweise größer null. In anderen Worten kann die Nutzlasteinrichtung in der Beschleunigungsphase im Betrieb sein, typischerweise mit verringerter Leistung. Alternativ oder zusätzlich kann in der Beschleunigungsphase die elektrische Versorgungsleistung gleich null sein.

Die aufnehmbare oder aufgenommene Drehfeldleistung, insbesondere die bereitstellbare oder bereitgestellte elektrische Versorgungsleistung, kann an einem Ausgang des Drehstrommotors für die Nutzlasteinrichtung anliegen. Der Ausgang des Drehstrommotors, insbesondere der Ausgang eines rotierenden Umrichters oder eines stationären Umrichters, ist mit der Nutzlasteinrichtung verbindbar. Am Ausgang liegt vorzugsweise eine Gleichspannung, alternativ eine Wechselspannung vor. Die Nutzlasteinrichtung kann alternativ oder zusätzlich einen Teil des Gantryantriebs bilden. Die Nutzlasteinrichtung kann insbesondere in eine Schaltungsanordnung, umfassend den Drehstrommotor, insbesondere den Drehstromstator und den Drehstromrotor, integriert sein.

Die Nutzlasteinrichtung ist insbesondere keine mechanische Antriebseinheit und/oder nicht zum Antreiben des Drehstromrotors geeignet. Die elektrische Versorgungsleistung wird in anderen Worten insbesondere nicht für die Drehbewegung des Drehstromrotors verwendet. Die Nutzlasteinrichtung kann drehfest mit dem Drehstromstator oder mit dem Drehstromrotor verbunden sein. Die Nutzlasteinrichtung kann derart elektrisch verschaltet sein, dass in einer Schaltungsanordnung der Drehstromstator zwischen dem Drehstromrotor und der Nutzlasteinrichtung ist oder dass der Drehstromrotor zwischen dem Drehstromstator und der Nutzlasteinrichtung ist.

Die Nutzlasteinrichtung kann eine Parallel- und/oder Reihenschaltung mehrerer elektrischer Nutzlasten vorsehen. Die elektrische Versorgungsleistung kann insbesondere für mehrere elektrische Nutzlasten eingesetzt werden. Eine elektrische Nutzlast kann beispielsweise als elektrischer Verbraucher benannt sein.

Es ist denkbar, dass die Nutzlasteinrichtung einen Hochspannungsgenerator und/oder einen Röntgenstrahler betreibbar mit einer elektrischen Versorgungsleistung größer 5 kW, insbesondere größer 50 kW, vorzugsweise größer 150 kW, aufweist. Der Hochspannungsgenerator und/oder der Röntgenstrahler ist typischerweise für die Bildgebung vorteilhaft und verbraucht im Betrieb die elektrische Versorgungsleistung. Im Röntgenstrahler werden Elektronen typischerweise mittels der Hochspannung auf Energien bis zu 150 keV beschleunigt. Ein Elektronenstrom beträgt beispielsweise zwischen 10 und 1000 mA, typischerweise in Abhängigkeit der Hochspannung bzw. der maximalen elektrischen Versorgungsleistung.

Die Regeleinheit ist insbesondere für eine Regelung des Drehstrommotors ausgebildet. Die Regelung des Drehstrommotors umfasst die Regelung des Drehstromstators und/oder die Regelung des Drehstromrotors. Die Regelung des Drehstromstators umfasst insbesondere die Regelung des stationären Umrichters. Die Regelung des Drehstromrotors umfasst insbesondere die Regelung des rotierenden Umrichters.

Die Regelziele der Regelung sind insbesondere die mechanische Antriebsleistung und/oder die elektrische Versorgungsleistung. Die Regelung umfasst insbesondere das Einstellen der Aufteilung der Drehfeldleistung zwischen mechanischer Antriebsleistung und elektrischer Versorgungsleistung. Die Regelung umfasst insbesondere ein Einprägen einer Spannung, insbesondere einer Amplitude und/oder eines Phasenwinkels, einer Frequenz und/oder eines Schlupfs und/oder eines Ausgangsstroms und/oder einer Ausgangsspannung für die Nutzlasteinrichtung, wovon die Drehfeldleistung typischerweise abhängt und/oder wodurch die Drehfeldleistung geregelt wird. Die Regelstellgrößen sind somit die Spannung, insbesondere die Amplitude und/oder des Phasenwinkels, die Frequenz und/oder des Schlupfs und/oder des Ausgangsstroms und/oder der Ausgangsspannung. Die Regelstellgrößen sind üblicherweise jeweils für den Drehstromstator und den Drehstromrotor verfügbar. Die Regelung ist insbesondere eine feldorientierte Regelung.

Die Regeleinheit ist typischerweise mit dem Drehstromstator verbunden. Zwischen der Regeleinheit und dem Drehstromstator kann der stationäre Umrichter verschaltet sein. Der stationäre Umrichter wird beispielsweise von dem, vorzugsweise dreiphasigen, Netzanschluss gespeist und/oder weist einen dreiphasigen Eingang auf. Grundsätzlich ist es denkbar, dass zwischen dem stationären Umrichter und dem Netzanschluss ein Zwischenkreis mit einer gleichgerichteten Netzspannung vorgesehen ist. In diesem Fall ist der stationäre Umrichter vorzugsweise zusätzlich in der Lage, in Art eines Wechselrichters Gleichspannung in Wechselspannung zu wandeln oder dem stationären Umrichter ist ein stationärer Wechselrichter vorgeschaltet. Der stationäre Umrichter kann insbesondere ein sogenannter indirekter Umrichter sein, welcher typischerweise eine Kombination aus einem Gleichrichter und einen Wechselrichter darstellt. Der stationäre Umrichter weist beispielsweise einen Kondensator auf.

Die Regeleinheit ist insbesondere zur Regelung des Drehstromstators, vorzugsweise des stationären Umrichters und/oder des stationären Wechselrichters, eingerichtet. Die Regelung ist insbesondere die Feldorientierte Regelung für eine Art Asynchronmaschine. Die Regelung des Drehstromstators umfasst insbesondere das Einprägen der Spannung, insbesondere der Amplitude und/oder des Phasenwinkels, und/oder der Frequenz.

Die Regeleinheit ist typischerweise mit dem Drehstromrotor verbunden. Zwischen der Regeleinheit und dem Drehstromrotor kann ein rotierender Umrichter verschaltet sein. Der rotierende Umrichter wird typischerweise von dem, insbesondere dreiphasigen, Drehstromrotor gespeist. Der rotierende Umrichter kann insbesondere einen rotierenden Gleichrichter umfassen, welcher insbesondere für die Nutzlasteinrichtung eine Gleichspannung mit der elektrischen Versorgungsleistung bereitstellt. Grundsätzlich ist es denkbar, dass zwischen dem Drehstromrotor und dem rotierenden Umrichter ein Zwischenkreis vorgesehen ist. In diesem Fall ist der rotierende Umrichter vorzugsweise zusätzlich in der Lage, in Art eines Wechselrichters Gleichspannung in Wechselspannung und/oder umgekehrt zu wandeln. Der rotierende Umrichter kann insbesondere ein Gleichrichter sein. Der rotierende Umrichter weist beispielsweise einen Kondensator auf.

Der Begriff "stationär" bezieht sich in diesem Kontext nur auf die Anordnung des einen Umrichters am Drehstromstator. Der stationäre Umrichter kann insbesondere erster Umrichter oder Statorumrichter genannt sein. Der Begriff "rotierend" bezieht sich in diesem Kontext nur auf die Anordnung des anderen Umrichters am Drehstromrotor. Der rotierende Umrichter kann insbesondere weiterer Umrichter oder zweiter Umrichter oder Rotorumrichter genannt sein. Der rotierende Umrichter kann grundsätzlich gleich wie der stationäre Umrichter oder verschieden aufgebaut sein.

Die Regeleinheit ist insbesondere zur Regelung des Drehstromrotors, vorzugsweise des rotierenden Umrichters eingerichtet. Die Regelung ist insbesondere die Feldorientierte Regelung für eine Art Asynchronmaschine. Die Regelung des Drehstromrotors umfasst insbesondere das Einprägen der Spannung, insbesondere der Amplitude und/oder des Phasenwinkels, und/oder der Frequenz.

Der Direktantrieb kann eine Sensoreinheit zur Messung der Drehgeschwindigkeit der Drehbewegung, insbesondere einer mechanischen Drehfrequenz des Drehstromrotors relativ zum Drehstromstator umfassen. Die Regeleinheit kann mit der Sensoreinheit zum Empfangen eines Messwerts der Sensoreinheit verbunden sein. Der Messwert kann zeitabhängig sein.

Die Regeleinheit kann insbesondere den ersten Anteil mittels der Regelung des Drehstromstators und den zweiten Anteil mittels der Regelung des Drehstromrotors variieren. Das Variieren umfasst insbesondere ein Erhöhen oder ein Verringern. Das Variieren eines der beiden Anteile kann derart erfolgen, dass beim Erhöhen der andere Anteil verringert und/oder die Drehfeldleistung erhöht wird, wobei beim Verringern der andere Anteil erhöht und/oder die Drehfeldleistung verringert wird. Für die Variation des ersten Anteils und/oder des zweiten Anteils verändert die Regeleinheit insbesondere einen Arbeitspunkt des Drehstromstators, insbesondere des stationären Umrichters, und/oder einen Arbeitspunkt des Drehstromrotors, insbesondere des rotierenden Umrichters.

Die Summe des ersten Anteils und des zweiten Anteils beträgt insbesondere kleiner oder gleich 100% der Drehfeldleistung, insbesondere kleiner oder gleich 100% einer Gesamtleistung der Drehfeldleistung. Absolut betrachtet kann die Gesamtleistung der Drehfeldleistung veränderbar sein, insbesondere bis hin zu einem Maximalwert. Der Maximalwert kann eine Leistungsreserve des Gantryantriebs ausnutzen.

Die Regeleinheit kann eine Recheneinheit umfassen, in welcher Programmcodemittel die feldoptimierte Regelung abbilden. Die Programmcodemittel können alternativ oder zusätzlich das Verändern des Arbeitspunkts des Drehstromstators und/oder des Arbeitspunkts des Drehstromrotors abbilden. Eine Beschreibung des Wirkprinzips lautet: Der Arbeitspunkt ist insbesondere eine Funktion der Drehstromfrequenz und gibt ein Drehmoment an. Die Drehmoment-Drehstromfrequenz-Charakteristik hängt von einem resistiven Wert des Drehstromrotors ab, wodurch typischerweise die Verluste im Rotor beeinflusst sind. Der rotierende Umrichter emuliert den resistiven Wert des Drehstromrotors insbesondere in der Fortlaufphase oder Volllastbetriebsphase. Das Drehmoment korreliert typischerweise mit der mechanischen Antriebsleistung. Das Drehmoment ist mittels der Drehfeldleistung bereitstellbar. Grundsätzlich sind alternative Betrachtungsweisen des Wirkprinzips denkbar.

Die Regeleinheit kann typischerweise die Arbeitspunkte wenigstens für folgende Szenarien regeln:

- maximaler magnetischer Fluss zur Maximierung der mechanischen Antriebsleistung, wenn z.B. in der Beschleunigungsphase ein maximales Drehmoment benötigt wird,
- ausgewogener magnetischer Fluss zur Erhöhung der elektrischen Versorgungsleistung im Vergleich zur mechanischen Antriebsleistung. In diesen beiden Szenarien wird der magnetische Fluss typischerweise zeitlich konstant gehalten. In anderen Worten sind diese Arbeitspunkte stationär. Der Drehstrommotor wird typischerweise nicht in Sättigung betrieben. Die Erhöhung der elektrischen Versorgungsleistung kann insbesondere dadurch erfolgen, dass der magnetische Fluss verringert und somit der Schlupf vergrößert wird.

Alternativ oder zusätzlich kann die Regeleinheit einen Arbeitspunkt für folgenden Szenario regeln:
- Modulation der magnetischen Flussamplitude, insbesondere wenn die mechanische Antriebsleistung null und die elektrische Versorgungsleistung größer null ist. Grundsätzlich ist bei diesem Szenario denkbar, dass die mechanische Antriebsleistung größer null ist. In diesem Szenario wirkt der Drehstrommotor als Transformator und ermöglicht vorzugsweise eine zeitliche Änderung des Flusses.

Grundsätzlich ist es denkbar, dass die Regeleinheit dazu ausgebildet ist, den ersten Anteil und/oder den zweiten Anteil durch Einstellen des Schlupfs des Gantryantriebs zu variieren. Die Drehstromfrequenz $f_{1el}$ des Drehstromstators (Statordrehfrequenz) abzüglich der Drehstromfrequenz $f_{2el}$ des Drehstromrotors (Rotordrehfrequenz) gibt typischerweise die mechanische Drehfrequenz $f_{mech}$ des Drehstromrotors an:

$$f_{mech} = (f_{1el} - f_{2el}) /$$

p; wobei p der Polpaarzahl entspricht.

Der Schlupf S des Gantryantriebs, insbesondere des Drehstrommotors, ergibt sich wie folgt:

$$S = (f_{1el} - f_{mech} \times p) / f_{1el} = f_{2el} / f_{1el}$$

Das Einstellen des Schlupfs ermöglicht somit vorteilhafterweise die Variation des ersten Anteils und/oder des zweiten Anteils. Zum Beispiel führt die Absenkung des magnetischen Flusses und/oder der Statordrehfrequenz zur Vergrößerung des Schlupfs. Die Drehbewegung, insbesondere die dafür nötige mechanische Antriebsleistung, kann typischerweise mit verschieden Regelstellgrößenkombinationen, insbesondere mit mehreren verschiedenen Kombinationen aus Statordrehfrequenz und Rotordrehfrequenz, erzeugt werden.

Die Regeleinheit kann eine Schnittstelle für einen Empfang eines Steuersignals aufweisen. Das Steuersignal kann zumindest ein Regelziel und/oder eine Regelstellgröße beschreiben. Die Regeleinheit ist vorzugsweise dazu ausgebildet, den ersten Anteil und/oder den zweiten Anteil in Abhängigkeit vom Steuersignal einzustellen. Das Steuersignal kann insbesondere einen Sollwert des ersten Anteils und/oder des zweiten Anteils umfassen. Das Steuersignal kann zeitabhängig sein. Das Steuersignal kann zusätzlich einen Istwert des ersten Anteils und/oder des zweiten Anteils umfassen. Das Steuersignal kann von einer Nutzung und/oder einem Nutzer des Gantryantriebs abhängen. Die Nutzung kann eine Bildgebung sein. Der Nutzer kann ein Arzt und/oder ein Betreiber des Gantryantriebs sein.

Das Steuersignal kann zumindest einen Wert für die Beschleunigungsphase und/oder einen Wert nach der Beschleunigungsphase umfassen, vorzugsweise jeweils für den ersten Anteil und/oder den zweiten Anteil. Der Wert für die Beschleunigungsphase ist beispielsweise die Sollgeschwindigkeit, insbesondere die Gantrydrehzahl und/oder die mechanische Drehfrequenz, und/oder ein Antriebsparameter. Von der Sollgeschwindigkeit hängt die mechanische Antriebsleistung bzw. Drehmoment ab. Der Wert nach der Beschleunigungsphase ist insbesondere ein Nutzlasteinrichtungsparameter, beispielsweise eine Zwischenkreisspannungsvorgabe am Ausgang des rotierenden Umrichters. Die Zwischenkreisspannungsvorgabe ist üblicherweise konstant. Der Nutzlasteinrichtungsparameter kann einen Zeitpunkt beim Wechsel in die Volllastbetriebsphase oder in die Beschleunigungsphase umfassen, so dass der Gantryantrieb einem Einbrechen oder Überschießen der Zwischenkreisspannung durch Anpassung der elektrischen Versorgungsleistung entgegenwirken kann. Der Wert für oder nach der Beschleunigungsphase kann insbesondere ein Lademanagementparameter für einen Energiepuffer sein. Der Lademanagementparameter kann insbesondere einen Beladestrom, einen Entladestrom, einen Zielwert des SoC-Werts. Der SoC-Wert gibt typischerweise das Ladelevel des Energiepuffers an.

Die Regeleinheit kann zur Regelung des Drehstromstators und/oder des Drehstromrotors kabelgebunden oder kabellos verbunden sein. In letzterem Fall sind in der Regelstrecke beispielsweise ein kabelloser Sender und ein kabelloser Empfänger vorgesehen, welche insbesondere optisch, kapazitiv oder induktiv ausgebildet sind. Eine kabelgebundene Verbindung kann eine Verwendung einer galvanischen oder resistiven Regelstrecke umfassen, z.B. in Form einer Bürste und eines Schleifrings.

Eine Ausführungsform des Gantryantriebs sieht vor, dass die Regeleinheit dazu ausgebildet ist, den ersten Anteil oder den zweiten Anteil auf Kosten des zweiten Anteils bzw. des ersten Anteils zu erhöhen. Diese Ausführungsform ist insbesondere vorteilhaft, weil somit die Drehfeldleistung nicht erhöht werden muss. Dies ist insbesondere vorteilhaft, wenn die Drehfeldleistung aufgrund fehlender Leistungsreserven nicht erhöht werden kann.

Dem erfindungsgemäßen Verfahren entsprechend sieht eine Ausführungsform in analoger Weise zur vorherigen Ausführungsform der Vorrichtung vor, dass der erste Anteil und der zweite Anteil mittels der Regeleinheit variiert wird. Weiterbildungen dieser Ausführungsform betreffen, dass mittels der Regeleinheit der erste Anteil derart eingestellt wird, dass in der Beschleunigungsphase des Drehstromrotors der erste Anteil im Vergleich zum ersten Anteil nach der Beschleunigungsphase erhöht ist, und/oder dass mittels der Regeleinheit der zweite Anteil derart eingestellt wird, dass nach der Beschleunigungsphase des Drehstromrotors der zweite Anteil im Vergleich zum zweiten Anteil in der Beschleunigungsphase erhöht ist.

Eine Ausführungsform des Gantryantriebs sieht vor, dass die Regeleinheit dazu ausgebildet ist, den ersten Anteil auf Kosten des zweiten Anteils in der Beschleunigungsphase des Drehstromrotors zu erhöhen. Auf Kosten bedeutet insbesondere, dass ein Leistungsdelta zwischen den beiden Anteilen in im Wesentlichen gleicher Höhe verschoben oder neu allokiert wird. In anderen Worten bedeutet die Erhöhung des ersten Anteils um das Leistungsdelta, dass gleichzeitig der zweite Anteil um das Leistungsdelta verringert ist. Diese Ausführungsform ist insbesondere vorteilhaft, weil in der Beschleunigungsphase auf elektrische Versorgungsleistung zumindest teilweise verzichtet werden kann, insbesondere wenn die Nutzlasteinrichtung für eine Bildgebung eingerichtet ist. Die Bildgebung wird vorzugsweise nicht in der Beschleunigungsphase, sondern erst nach Erreichen der Sollgeschwindigkeit durchgeführt, um von der erhöhten Drehbewegung zeitlich zu profitieren.

Dem erfindungsgemäßen Verfahren entsprechend sieht eine Ausführungsform in analoger Weise zur vorherigen Ausführungsform der Vorrichtung vor, dass in der Beschleunigungsphase des Drehstromrotors der erste Anteil auf Kosten des zweiten Anteils mittels der Regeleinheit erhöht wird.

Eine Ausführungsform des Gantryantriebs sieht vor, dass die Regeleinheit dazu ausgebildet ist, den zweiten Anteil auf Kosten des ersten Anteils nach der Beschleunigungsphase des Drehstromrotors zu erhöhen. Typischerweise benötigt die Nutzlasteinrichtung in der Fortlaufphase, insbesondere nach Erreichen der Sollgeschwindigkeit, mehr elektrische Versorgungsleistung und der Drehstromrotor weniger mechanische Antriebsleistung zur Aufrechterhaltung der Sollgeschwindigkeit. Somit kann ein weiteres Leistungsdelta zum Vorteil des zweiten Anteils auf Kosten des ersten Anteils verschoben werden, ohne dass eine Leistungsreserve vorgesehen oder abgerufen werden muss.

Dem erfindungsgemäßen Verfahren entsprechend sieht eine Ausführungsform in analoger Weise zur vorherigen Ausführungsform der Vorrichtung vor, dass nach der Beschleunigungsphase des Drehstromrotors der zweite Anteil auf Kosten des ersten Anteils mittels der Regeleinheit erhöht wird.

Eine Ausführungsform des Gantryantriebs sieht vor, dass die Regeleinheit dazu ausgebildet ist, den Drehstrommotor als Transformator zur Modulation des magnetischen Flusses anzusteuern, um den ersten Anteil und/oder den zweiten Anteil zu variieren. Diese Ausführungsform ist insbesondere im Stillstand des Gantryantriebs vorteilhaft, um die Nutzlasteinrichtung mit dem zweiten Anteil der Drehfeldleistung betreiben zu können.

Eine Ausführungsform des Gantryantriebs sieht vor, dass die Regeleinheit dazu ausgebildet ist, den ersten Anteil und/oder den zweiten Anteil gemäß einer feldorientierten Regelung zu variieren. Die feldorientierte Regelung umfasst insbesondere untergelagerte Regelungen, welche zumindest eine der Regelstellgrößen in d- und q-Koordinaten abbildet. Die untergelagerten Regelungen sind insbesondere Maschinengleichungen des Drehstrommotors in feldorientierten Koordinaten. Bei der feldorientierten Regelung ist der Schlupf typischerweise keine direkte Regelstellgröße. Der Schlupf wird üblicherweise bei der feldorientierten Regelung verändert.

Dem erfindungsgemäßen Verfahren entsprechend sieht eine Ausführungsform in analoger Weise zur vorherigen Ausführungsform der Vorrichtung vor, dass der erste Anteil und der zweite Anteil gemäß der feldorientierten Regelung mittels der Regeleinheit variiert wird.

Eine Ausführungsform sieht vor, dass der Gantryantrieb einen mit dem Drehstrommotor elektrisch verschalteten Energiepuffer aufweist, wobei die Regeleinheit dazu ausgebildet ist, den Energiepuffer in Abhängigkeit von der bereitstellbaren Drehfeldleistung zu entladen oder aufzuladen. Dass der Energiepuffer mit dem Drehstromrotor elektrisch verschaltet ist, bedeutet insbesondere, dass der Energiepuffer und der Drehstromrotor gemeinsam eine Schaltungsanordnung oder einen Teil einer Schaltungsanordnung bilden. Der Energiepuffer kann insbesondere Teil des Gantryantriebs oder einer Computertomographieeinrichtung sein.

Der Energiepuffer kann mit dem Drehstromstator, dem stationären Umrichter, mit dem Drehstromrotor oder mit dem rotierenden Umrichter elektrisch verschaltet sein. Der Energiepuffer kann drehfest mit dem Drehstromstator oder mit dem Drehstromrotor verbunden sein. Der Energiepuffer am Drehstromstator bietet den Vorteil, dass weniger Masse rotiert und somit eine Antriebsleistung geringer sein kann. Außerdem ist üblicherweise am Drehstromstator mehr Bauraum verfügbar als am Drehstromrotor.

Der Energiepuffer am Drehstromrotor bietet den Vorteil, dass insgesamt der Drehstrommotor kleiner oder leistungsschwächer ausfallen kann, insbesondere wenn der Energiepuffer mit dem rotierenden Umrichter und/oder dem Drehstromrotor elektrisch verschaltet ist und/oder weitere mechanische Antriebsleistung und/oder elektrische Versorgungsleistung bereitstellt. Beispielsweise kann beim Betrieb einer Nutzlasteinrichtung, welche in der Spitze Anforderungen größer 5 kW oder 50 kW oder 150 kW erfordert, die Drehfeldleistung um mindestens den Faktor 2, 3 oder 5 oder 10 kleiner ausfallen, wenn der Energiepuffer zur Leistungskompensation am Drehstromrotor vorgesehen ist.

Der Energiepuffer kann eine Batterie und/oder ein Akkumulator und/oder ein Kondensator, insbesondere ein Superkondensator und/oder ein Doppelschichtkondensator, sein. Der Energiepuffer speichert insbesondere elektrische Energie. Der Energiepuffer kann typischerweise aus mehreren Energiepufferzellen aufgebaut sein, welche vorzugsweise zumindest teilweise ringförmig den Hohlraum räumlich umgeben. Der Energiepuffer ist mit dem Drehstromrotor derart elektrisch verschaltet, dass die bereitstellbare Drehfeldleistung den Energiepuffer aufladen kann. Der Energiepuffer bietet den Vorteil, dass die Leistungsreserve des Drehstrommotors, insbesondere des Drehstromrotors und/oder des Drehstromstators, geringer aus- oder komplett entfallen kann. Für den Betrieb des Direktantriebs unter Volllast und/oder Spitzenlast, wie z.B. in der Beschleunigungsphase, kann der Energiepuffer vorteilhafterweise die Leistungsreserve zumindest teilweise kompensieren. Das ermöglicht vorteilhafterweise Kostenvorteile, insbesondere durch eine Einsparung von Material bei den Drehstromwicklungen. Alternativ oder zusätzlich ist der Bauraum nahe dem Hohlraum reduziert, so dass der Hohlraum größer sein kann.

Eine Ausführungsform sieht vor, dass der Energiepuffer mit dem Drehstrommotor verschaltet und mittels der Regeleinheit derart regelbar ist, dass zusätzlich zum ersten Anteil eine weitere mechanische Antriebsleistung zur Erzeugung der Drehbewegung des Drehstromrotors mittels Energie aus dem Energiepuffer bereitstellbar ist. Die weitere mechanische Antriebsleistung in Summe mit dem ersten Anteil und dem zweiten Anteil übersteigt typischerweise die aufnehmbare oder aufgenommene Drehfeldleistung. In anderen Worten kann die weitere mechanische Antriebsleistung mittels der Drehfeldleistung zumindest an einem Zeitpunkt des Betriebs des Gantryantriebs nicht bereitstellbar sein. Es ist grundsätzlich denkbar, dass eine Gesamtleistung der Drehfeldleistung zu einem anderen Zeitpunkt des Betriebs des Gantryantriebs einer Summe der weiteren mechanischen Antriebsleistung, der mechanischen Antriebsleistung und der elektrischen Versorgungsleistung entsprechen kann. Durch das Bereitstellen der weiteren mechanischen Antriebsleistung wird der Energiepuffer typischerweise entladen. Insbesondere in der Beschleunigungsphase ist diese Ausführungsform vorteilhaft und/oder zur Reduktion der Leistungsreserve des Drehstromrotors und/oder des Drehstromstators.

Eine Ausführungsform sieht vor, dass der Energiepuffer mit dem Drehstrommotor verschaltet und mittels der Regeleinheit derart regelbar ist, dass im Generatorbetrieb des Gantryantriebs der Energiepuffer aufladbar ist. Der Energiepuffer kann insbesondere die kinetische Energie des Drehstromrotors in Form elektrischer Energie speichern, wobei der Energiepuffer aufgeladen wird. Der Energiepuffer kann vorzugsweise derart ein Abbremsen des Drehstromrotors verstärken. Das Aufladen des Energiepuffers erfolgt insbesondere durch Wandlung des Trägheitsmoments des sich rotierenden Drehstromrotors.

Eine Ausführungsform sieht vor, dass der Energiepuffer mit der Nutzlasteinrichtung derart verschaltet und mittels der Regeleinheit derart regelbar ist, dass zusätzlich zum zweiten Anteil eine weitere elektrische Versorgungsleistung der Nutzlasteinrichtung mittels Energie aus dem Energiepuffer bereitstellbar ist. Die weitere elektrische Versorgungsleistung in Summe mit dem ersten Anteil und dem zweiten Anteil übersteigt typischerweise die aufnehmbare oder aufgenommene Drehfeldleistung. Durch das Bereitstellen der weiteren elektrischen Versorgungsleistung wird der Energiepuffer typischerweise entladen. Insbesondere nach der Beschleunigungsphase und/oder in der Volllastbetriebsphase ist diese Ausführungsform vorteilhaft und/oder zur Reduktion der Leistungsreserve des Drehstrommotors, insbesondere des Drehstromrotors und/oder des Drehstromstators. Ein weiterer Vorteil dazu ist, dass die aus dem Netz aufgenommene Spitzenleistung vorzugsweise sinkt. Dadurch wird typischerweise eine geringere Netzanschlussleistung benötigt. Das ist insbesondere vorteilhaft für kleiner dimensionierte Netzanschlüsse, welche zum Betrieb des erfindungsgemäßen Gantryantriebs vorgesehen sind.

Eine Ausführungsform sieht vor, dass der Energiepuffer derart mit dem Drehstrommotor und der Nutzlasteinrichtung verschaltet ist, dass die Drehfeldleistung ausschließlich mittels Energie aus dem Energiepuffer bereitstellbar ist. Der Energiepuffer wirkt in diesem Fall vorteilhafterweise als unterbrechungsfreie Stromversorgung. In anderen Worten funktioniert der Gantryantrieb vorzugsweise ohne Netzanschlussleistung, wenn das Ladelevel des Energiepuffers größer null ist. Ein solcher Energiepuffer ist insbesondere vorteilhaft, wenn beispielsweise das Netz ausfällt. Diese Ausführungsform wird insbesondere dadurch ermöglicht, dass der Aufbau des Drehstrommotors symmetrisch ist. Die Regeleinheit ist vorzugsweise dazu ausgebildet, den Netzausfall zu erkennen und den Energiepuffer entsprechend anzusteuern. In einer ersten Betrachtung ersetzt in dieser Ausführungsform die weitere mechanische Antriebsleistung den ersten Anteil und/oder die weitere elektrische Versorgungsleistung den zweiten Anteil in Bezug auf die übliche Übertragungsrichtung der Drehfeldleistung vom Drehstromstator auf den Drehstromrotor. In einer zweiten Betrachtung kehrt sich die Richtung der Drehfeldleistung insgesamt um, so dass nun die Drehfeldleistung vom Drehstromrotor an den Drehstromstator übertragen wird.

Eine alternative Ausführungsform sieht vor, dass der Drehstromrotor ausschließlich mit der bereitgestellten mechanischen Antriebsleistung betreibbar ist. Diese Ausführungsform ist insbesondere vorteilhaft, weil dafür kein Energiepuffer benötigt ist.

Eine weitere alternative Ausführungsform sieht vor, dass die Nutzlasteinrichtung ausschließlich mit der bereitgestellten elektrischen Versorgungsleistung betreibbar ist. Diese Ausführungsform ist insbesondere vorteilhaft, weil dafür kein Energiepuffer benötigt ist.

Die erfindungsgemäße Computertomographieeinrichtung weist

- den Gantryantrieb,
- einen stationären Trägerring und
- einen rotierenden Trägerring auf,
- wobei der stationäre Trägerring mit dem Drehstromstator verbunden ist und der rotierende Trägerring drehfest relativ zum Drehstromrotor angeordnet ist. In anderen Worten dreht sich der rotierende Trägerring gemeinsam mit dem Drehstromrotor relativ zum Drehstromstator bzw. dem stationären Trägerring. Der rotierende Trägerring und/oder der stationäre Trägerring kann eine zentrale Ausnehmung für den Hohlraum aufweisen. Eine äußere Form des stationären Trägerrings und/oder eine äußere Form des rotierenden Trägerrings kann trommelförmig oder scheibenförmig sein.

Die Computertomographieeinrichtung weist den erfindungsgemäßen Gantryantrieb auf und teilt sich somit die im Zusammenhang mit dem Gantryantrieb zuvor erwähnten Vorteile und umfasst dessen Ausführungsformen. Die Computertomographieeinrichtung kann aufgrund des verringerten Bauraums vorzugsweise leichter sein, so dass die mechanische Antriebsleistung zur Erreichung der Sollgeschwindigkeit des Drehstromrotors geringer ausfallen kann.

Die Computertomographieeinrichtung kann einen Erdungsschleifring umfassen, der aus regulatorischen, insbesondere zur Gewährung eines sicheren Betriebs, und/oder operativen Gründen vorzusehen sein kann.

Eine Ausführungsform sieht vor, dass die Computertomographieeinrichtung ferner einen Röntgendetektor aufweist, wobei mittels der Drehfeldleistung die elektrische Versorgungsleistung dem Röntgenstrahler der Nutzlasteinrichtung bereitstellbar ist. Vorzugsweise umfasst die Nutzlasteinrichtung den Röntgenstrahler, wenn vorzugsweise am rotierenden Trägerring der Röntgendetektor der Computertomographieeinrichtung drehfest angeordnet ist. Der Röntgendetektor und der Röntgenstrahler sind insbesondere für die Bildgebung ausgebildet und üblicherweise gegenüber voneinander am rotierenden Trägerring angeordnet.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt, insbesondere nicht nur in den zuvor explizit hervorgehobenen Fällen. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleichbleibende Strukturen und Einheiten mit demselben Bezugszeichen wie beim erstmaligen Auftreten der jeweiligen Struktur oder Einheit benannt.

Es zeigen:

Fig. 1 eine herkömmliche Gantry,

Fig. 2 einen erfindungsgemäßen Gantryantrieb,

Fig. 3 den Gantryantrieb in einem ersten Ausführungsbeispiel,

Fig. 4 den Gantryantrieb in einem zweiten Ausführungsbeispiel,

Fig. 5 eine erfindungsgemäße Computertomographieeinrichtung,

Fig. 6 die Computertomographieeinrichtung in einem ersten Ausführungsbeispiel,

Fig. 7 ein erfindungsgemäßes Verfahren,

Fig. 8 einen Leistungsfluss im Gantryantrieb bei einer ersten Betriebsart,

Fig. 9 einen Leistungsfluss im Gantryantrieb bei einer zweiten Betriebsart,

Fig. 10 einen stationären Umrichter und

Fig. 11 einen rotierenden Umrichter.

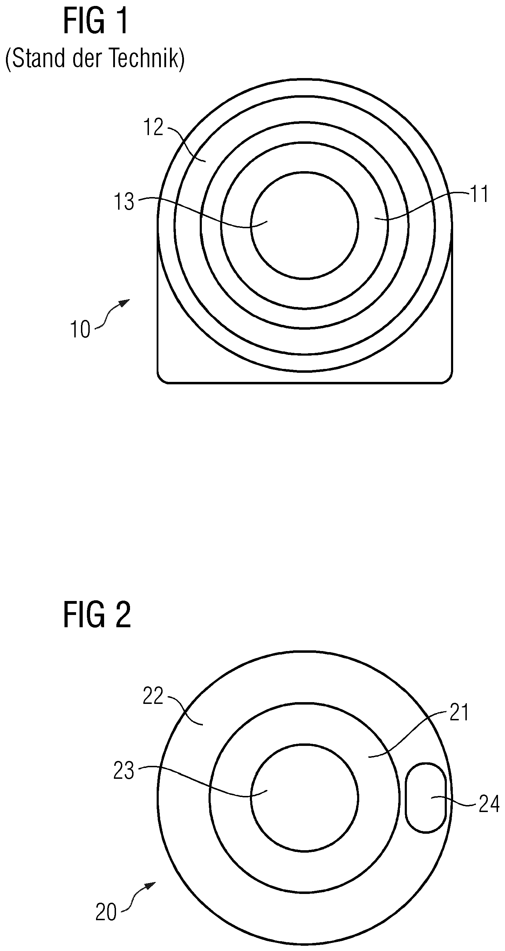

Fig. 1 zeigt eine schematische Ansicht einer herkömmlichen Gantry 10. Die herkömmliche Gantry 10 weist einen Gantryantrieb 11 als mechanischen Antrieb für einen rotierenden Trägerring auf und zusätzlich ein in diesem Ausführungsbeispiel kontaktbehaftetes Leistungsübertragungssystem 12. Das kontaktbehaftete Leistungsübertragungssystem 12 ist an einem Rotor angeordnet, welcher einen tunnelförmigen Hohlraum 13 zur Aufnahme eines Patienten räumlich umgibt.

Fig. 2 zeigt eine schematische Ansicht eines erfindungsgemäßen Gantryantriebs 20.

Der Gantryantrieb 20 weist einen Drehstromstator 21, einen Drehstromrotor 22, einen tunnelförmigen Hohlraum 23 zur Aufnahme eines Patienten und eine Regeleinheit 24 auf. Der Drehstromstator 21 und der Drehstrommotor 22 bilden einen Drehstrommotor zum Bereitstellen einer Drehfeldleistung. Dargestellt ist ein Drehstrommotor mit Außenläufer. Alternativ ist eine Ausführung des Drehstrommotors mit Innenläufer denkbar.

Der Drehstromrotor 22 umgibt den Hohlraum 23 räumlich. Zusätzlich umgibt der Drehstromstator 21 den Hohlraum 23 räumlich in diesem Ausführungsbeispiel. In diesem Ausführungsbeispiel wird die Drehfeldleistung im Wesentlichen in radialer Richtung übertragen, was üblicherweise mit einer trommelförmigen Ausgestaltung des Drehstromstators 21 und des Drehstromrotors 22 einhergeht. Alternativ kann die Drehfeldleistung im Wesentlichen in axialer Richtung übertragen werden, insbesondere dann sind der Drehstromstator 21 und der Drehstromrotor 22 scheibenförmig ausgebildet.

Mittels der Drehfeldleistung ist einerseits, in Form eines ersten Anteils, eine mechanische Antriebsleistung zur Erzeugung einer Drehbewegung des Drehstromrotors 22 und andererseits, in Form eines zweiten Anteils, eine elektrische Versorgungsleistung einer Nutzlasteinrichtung 25 bereitstellbar. Die Nutzlasteinrichtung 25 ist in Fig. 2 nicht gezeigt. Grundsätzlich kann die Nutzlasteinrichtung 25 drehfest mit dem Drehstromstator 21 oder mit dem Drehstromrotor 22 verbunden sein.

Die Regeleinheit 24 ist dazu ausgebildet ist, den ersten Anteil und den zweiten Anteil zu variieren. In Fig. 2 ist die Regeleinheit 24 auf dem rotierenden Teil dargestellt. Alternativ kann die Regeleinheit 24 stationär angeordnet sein, so dass die Regeleinheit 24 sich nicht mit dem Drehstromrotor 22 mit dreht.

Die Regeleinheit 24 ist vorzugsweise dazu ausgebildet, den ersten Anteil oder den zweiten Anteil auf Kosten des zweiten Anteils bzw. des ersten Anteils zu erhöhen. Die Regeleinheit 24 ist ferner vorzugsweise dazu ausgebildet, den ersten Anteil auf Kosten des zweiten Anteils in einer Beschleunigungsphase des Drehstromrotors 22 zu erhöhen. Weiterhin ist die Regeleinheit 24 vorzugsweise dazu ausgebildet, den zweiten Anteil auf Kosten des ersten Anteils nach der Beschleunigungsphase des Drehstromrotors 22 zu erhöhen. Die Regeleinheit 24 ist vorzugsweise dazu ausgebildet, den ersten Anteil und/oder den zweiten Anteil durch Einstellen des Schlupfs des Gantryantriebs 20 zu variieren. Alternativ oder zusätzlich ist die Regeleinheit 24 dazu ausgebildet, den Drehstrommotor als Transformator zur Modulation des magnetischen Flusses anzusteuern, um den ersten Anteil und/oder den zweiten Anteil zu variieren.

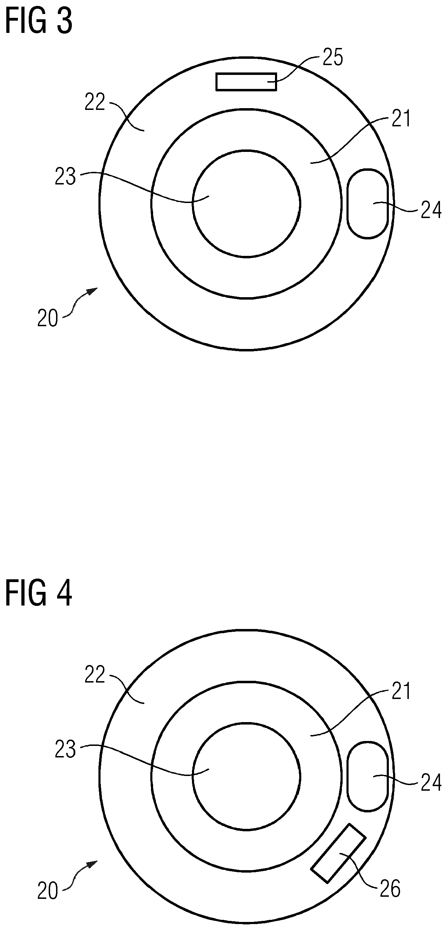

Fig. 3 zeigt den Gantryantrieb 20 der Fig. 2 in einem ersten Ausführungsbeispiel. In diesem Ausführungsbeispiel ist die Nutzlasteinrichtung 25 am Drehstromrotor 22 drehfest angeordnet.

Die Nutzlasteinrichtung 25 weist insbesondere einen Hochspannungsgenerator und/oder einen Röntgenstrahler auf. Dieser ist oder diese sind vorzugsweise mit einer elektrischen Versorgungsleistung größer 5 kW, insbesondere größer 50 kW, besonders vorteilhafterweise größer 150 kW betreibbar, welche vom Gantryantrieb 20 bereitstellbar ist.

Fig. 4 zeigt den Gantryantrieb 20 der Fig. 2 in einem zweiten Ausführungsbeispiel.

Der Gantryantrieb 20 weist einen mit dem Drehstrommotor elektrisch verschalteten Energiepuffer 26 auf, welcher mit dem Drehstromrotor 22 drehfest verbunden ist. Alternativ kann der Energiepuffer 26 mit dem Drehstromstator 21 drehfest verbunden sein. Die Regeleinheit 24 ist dazu ausgebildet ist, den Energiepuffer 26 in Abhängigkeit von der bereitstellbaren Drehfeldleistung zu entladen oder aufzuladen.

Vorzugsweise ist der Energiepuffer 26 mit dem Drehstrommotor verschaltet und mittels der Regeleinheit 24 derart regelbar, dass zusätzlich zum ersten Anteil eine weitere mechanische Antriebsleistung zur Erzeugung der Drehbewegung des Drehstromrotors 22 mittels Energie aus dem Energiepuffer 26 bereitstellbar ist. Alternativ oder zusätzlich ist der Energiepuffer 26 mit dem Drehstrommotor verschaltet und mittels der Regeleinheit 24 derart regelbar, dass im Generatorbetrieb des Gantryantriebs 20 der Energiepuffer 26 aufladbar ist. Vorteilhafterweise ist der Energiepuffer 26 mit der Nutzlasteinrichtung 25 derart verschaltet und mittels der Regeleinheit 24 derart regelbar, dass zusätzlich zum zweiten Anteil eine weitere elektrische Versorgungsleistung der Nutzlasteinrichtung 25 mittels Energie aus dem Energiepuffer 26 bereitstellbar ist. Alternativ oder zusätzlich ist der Energiepuffer 26 derart mit dem Drehstrommotor und der Nutzlasteinrichtung 25 verschaltet, dass die Drehfeldleistung ausschließlich mittels Energie aus dem Energiepuffer 26 bereitstellbar ist.

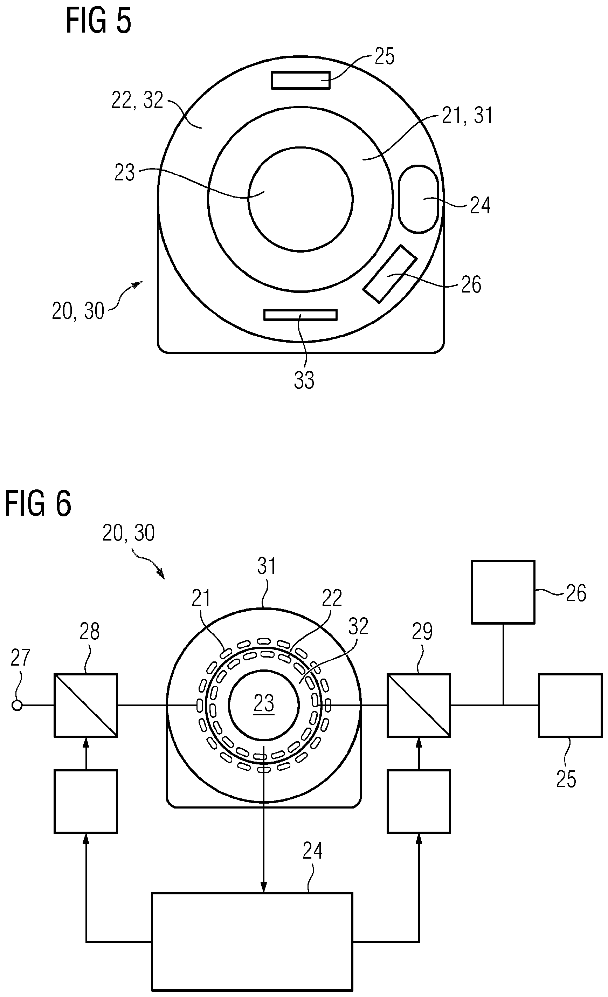

Fig. 5 zeigt eine schematische Ansicht einer erfindungsgemäßen Computertomographieeinrichtung 30.

Die Computertomographieeinrichtung 30 weist den Gantryantrieb 20, einen stationären Trägerring 31 und einen rotierenden Trägerring 32 auf. Der stationäre Trägerring 31 ist mit dem Drehstromstator 21 verbunden. Der rotierende Trägerring 32 ist drehfest relativ zum Drehstromrotor 22 angeordnet.

Der stationäre Trägerring 31 und/oder der rotierende Trägerring 32 können aus Metall, beispielsweise Aluminium, oder einem Kunststoff bestehen. Bei einer Ausführung aus Metall kann der stationäre Trägerring und/oder der rotierende Trägerring als elektrische Masseverbindung vorgesehen sein. Es ist denkbar, den Kunststoff mit zusätzlichen, insbesondere metallischen, Einlagen oder Strukturen zu verstärken. Eine äußere Form des stationären Trägerrings 31 und/oder des rotierenden Trägerrings 32 kann trommel- oder scheibenförmig sein.

Der stationäre Trägerring 31 weist typischerweise eine drehfeste Verbindung mit dem Drehstromstator 31 auf. Der rotierende Trägerring 32 weist typischerweise eine drehfeste Verbindung mit dem Drehstromrotor 32 auf. Der stationäre Trägerring 31 ist über eine Basis typischerweise mit oder auf einem Boden befestigbar.

Vorzugsweise weist die Computertomographieeinrichtung 30 einen Röntgendetektor 33 auf. Mittels der Drehfeldleistung ist die elektrische Versorgungsleistung vorteilhafterweise einem Röntgenstrahler der Nutzlasteinrichtung 25 bereitstellbar. Alternativ oder zusätzlich ist mittels der Drehfeldleistung die elektrische Versorgungsleistung dem Röntgendetektor 33 bereitstellbar.

Fig. 6 zeigt einen schematischen Regelkreis der Computertomographieeinrichtung 30 in einem ersten Ausführungsbeispiel. Im Vergleich zum vorherigen Ausführungsbeispiel ist der Drehstromrotor 22 in diesem Ausführungsbeispiel ein Innenläufer.

Die Drehstromwicklungen des Drehstromstators 21 und die Drehstromwicklungen des Drehstromrotors 22 sind als ellipsenförmige Einheiten, welche ringförmig um den Hohlraum 23 herum angeordnet sind, dargestellt. Die Anzahl der gezeigten Drehstromwicklungen dient nur illustratorischer Zwecke.

Die Regeleinheit 24 regelt einen stationären Umrichter 28 und einen rotierenden Umrichter 29 in Abhängigkeit einer mechanischen Drehfrequenz des Drehstromrotors 22. Die Regelung des stationären Umrichters 28 und des rotierenden Umrichters 29 erfolgt insbesondere durch ein Einstellen einer Regelstellgröße, vorzugsweise durch ein Einprägen einer Spannung, insbesondere einer Amplitude und/oder eines Phasenwinkels, einer Frequenz und/oder eines Schlupfs und/oder eines Ausgangsstroms und/oder einer Ausgangsspannung für die Nutzlasteinrichtung 25.

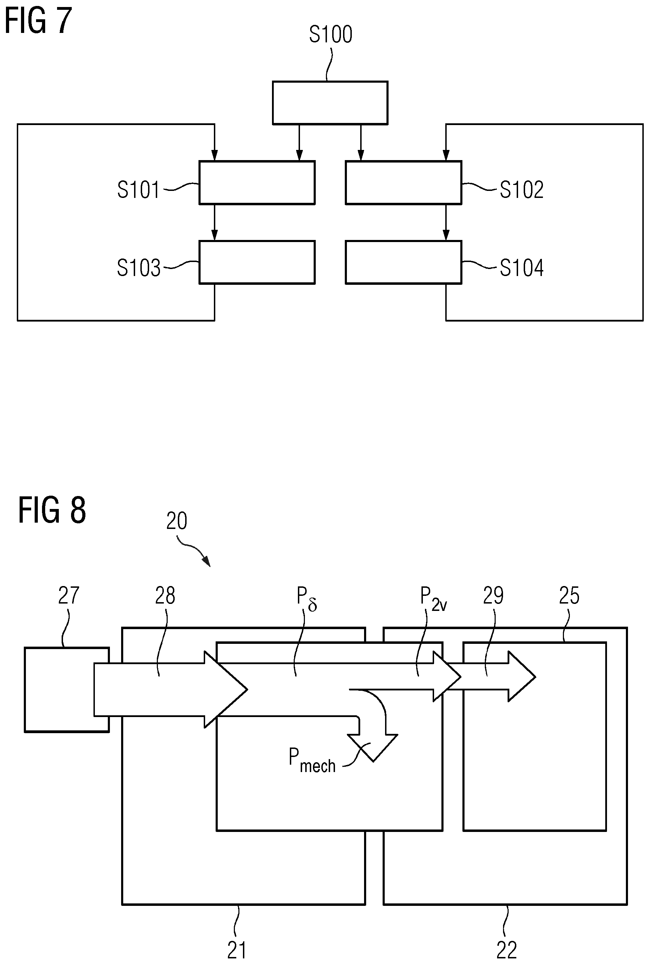

Fig. 7 zeigt ein erfindungsgemäßes Verfahren zum Erzeugen einer Drehbewegung eines Drehstromrotors 22 mittels eines Gantryantriebs 20 in einem Flussdiagramm mit den Verfahrensschritten S100 bis S102:
Verfahrensschritt S100 kennzeichnet ein Bereitstellen einer Drehfeldleistung in einem Drehstrommotor.

Verfahrensschritt S101 kennzeichnet, dass einerseits ein erster Anteil der Drehfeldleistung als mechanische Antriebsleistung zur Erzeugung der Drehbewegung des Drehstromrotors 22 des Drehstrommotors wirkt.

Verfahrensschritt S102 kennzeichnet, dass andererseits ein zweiter Anteil der Drehfeldleistung als elektrische Versorgungsleistung der Nutzlasteinrichtung 25 wirkt.

Ferner zeigt Fig. 7 mögliche Weiterbildungen mit den Verfahrensschritten S103 und S104:
Der optionale Verfahrensschritt S103 kennzeichnet, dass mittels einer Regeleinheit 24 der erste Anteil derart eingestellt wird, dass in einer Beschleunigungsphase des Drehstromrotors 22 der erste Anteil im Vergleich zum ersten Anteil nach der Beschleunigungsphase erhöht ist.

Der optionale Verfahrensschritt S104 kennzeichnet, dass mittels einer Regeleinheit 24 der zweite Anteil derart eingestellt wird, dass nach einer Beschleunigungsphase des Drehstromrotors 22 der zweite Anteil im Vergleich zum zweiten Anteil in der Beschleunigungsphase erhöht ist.

Fig. 8 zeigt in einem Flussdiagramm den Fluss der Leistungen im erfindungsgemäßen Gantryantrieb 20 bei einer ersten Betriebsart.

Der Drehstromstator 21 stellt insbesondere die Drehfeldleistung $P_\delta$ über den Luftspalt bereit, welche sich aufteilt in den ersten Anteil, der mechanischen Antriebsleistung $P_{mech}$, und den zweiten Anteil, der elektrischen Versorgungs-leistung $P_{2v}$. An den dreiphasigen Netzanschluss 27 mit einer Eingangsleistung ist ein stationärer Umrichter 28 angeschlossen. Aus der Eingangsleistung wird die Drehfeldleistung $P_\delta$ erzeugt, welche einerseits die mechanische Antriebsleistung $P_{mech}$ zur Erzeugung der Drehbewegung des Drehstromrotors 22 und andererseits die elektrische Versorgungs-leistung $P_{2v}$ der Nutzlasteinrichtung 25 bereitstellt.

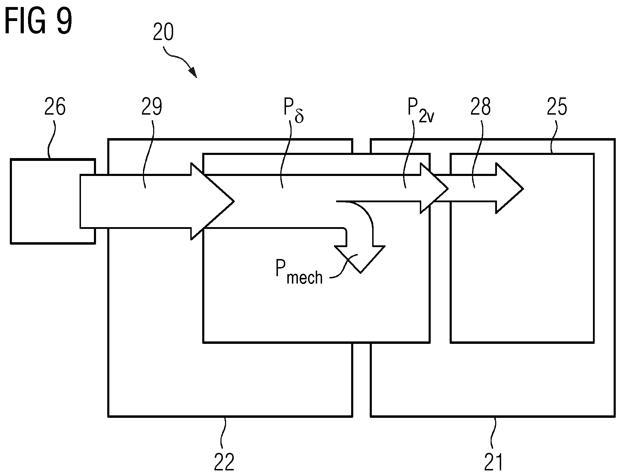

Fig. 9 zeigt in einem Flussdiagramm den Fluss der Leistungen im Gantryantrieb 20 bei einer zweiten Betriebsart. Im Vergleich zum in Fig. 8 gezeigten Ausführungsbeispiel kehrt sich die Richtung der Drehfeldleistung als Erklärungsmodell insgesamt um, so dass nun die Drehfeldleistung vom Drehstromrotor 22 an den Drehstromstator 21 übertragen wird. Weiterhin ist in diesem Ausführungsbeispiel die Nutzlasteinrichtung 25 statorseitig vorgesehen, wobei die Nutzlasteinrichtung der Fig. 8 rotorseitig vorgesehen ist.

Der Gantryantrieb 20 weist den mit dem Drehstrommotor elektrisch verschalteten Energiepuffer 26 auf. Der Energiepuffer 26 ist derart mit dem Drehstrommotor und der Nutzlasteinrichtung 25 verschaltet, dass die Drehfeldleistung ausschließlich mittels Energie aus dem Energiepuffer 26 bereitstellbar ist.

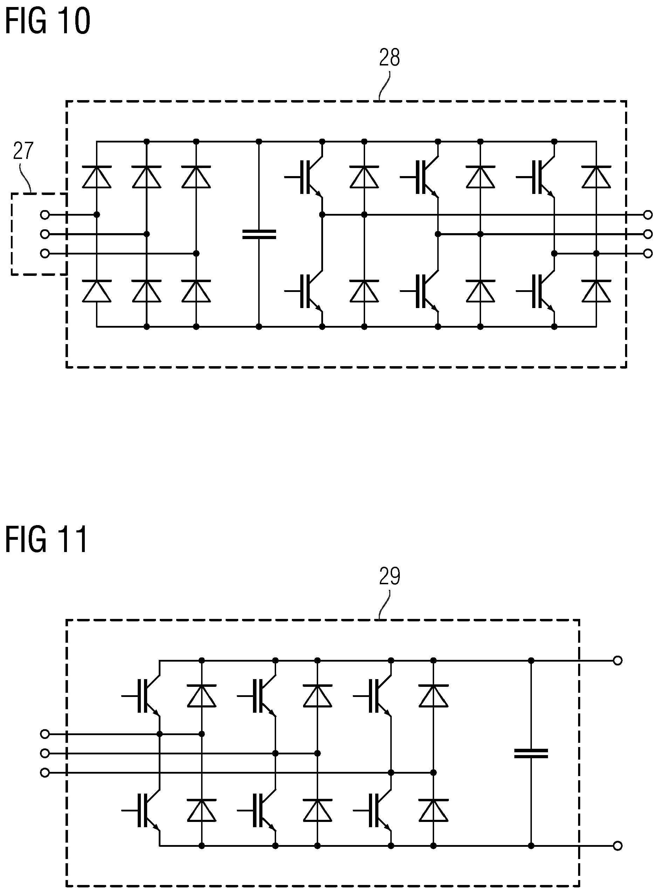

Fig. 10 zeigt eine Schaltungsanordnung des stationären Umrichters 28.

Fig. 11 zeigt eine Schaltungsanordnung des rotierenden Umrichters 29. Aus den in den Fig. 10 und 11 dargestellten Schaltungsanordnungen ist die Symmetrie des Drehstrommotors zu erkennen.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

**Patentansprüche**

1. Gantryantrieb (20), aufweisend

   - einen Drehstromstator (21),
   - einen Drehstromrotor (22) und
   - einen tunnelförmigen Hohlraum (23) zur Aufnahme eines Patienten
   - wobei der Drehstromrotor (22) den Hohlraum (23) räumlich umgibt,
   - wobei der Drehstromstator (21) und der Drehstromrotor (22) einen Drehstrommotor zum Bereitstellen einer Drehfeldleistung bilden,
   - wobei mittels der Drehfeldleistung einerseits, in Form eines ersten Anteils, eine mechanische Antriebsleistung zur Erzeugung einer Drehbewegung des Drehstromrotors (22) und andererseits, in Form eines zweiten Anteils, eine elektrische Versorgungsleistung einer Nutzlasteinrichtung (25) bereitstellbar ist,

   **dadurch gekennzeichnet, dass** der Gantryantrieb eine Regeleinheit (24) umfasst, welche dazu ausgebildet ist, den ersten Anteil und den zweiten Anteil zu variieren.

2. Gantryantrieb (20) nach Anspruch 1,
   wobei die Regeleinheit (24) dazu ausgebildet ist, den ersten Anteil oder den zweiten Anteil auf Kosten des zweiten Anteils bzw. des ersten Anteils zu erhöhen.

3. Gantryantrieb (20) nach Anspruch 2,
   wobei die Regeleinheit (24) dazu ausgebildet ist, den ersten Anteil auf Kosten des zweiten Anteils in einer Beschleunigungsphase des Drehstromrotors (22) zu erhöhen und/oder den zweiten Anteil auf Kosten des ersten Anteils nach der Beschleunigungsphase des Drehstromrotors (22) zu erhöhen.

4. Gantryantrieb (20) nach einem der vorhergehenden Ansprüche,
   wobei die Regeleinheit (24) dazu ausgebildet ist, den Drehstrommotor als Transformator zur Modulation des magnetischen Flusses anzusteuern, um den ersten Anteil und/oder den zweiten Anteil zu variieren.

5. Gantryantrieb (20) nach einem der vorhergehenden Ansprüche,
   wobei die Regeleinheit (24) dazu ausgebildet ist, den ersten Anteil und/oder den zweiten Anteil gemäß einer feldorientierten Regelung zu variieren.

6. Gantryantrieb (20) nach einem der vorhergehenden Ansprüche,
   wobei der Gantryantrieb (20) einen mit dem Drehstrommotor elektrisch verschalteten Energiepuffer (26) aufweist, wobei die Regeleinheit (24) dazu ausgebildet ist, den Energiepuffer (26) in Abhängigkeit von der bereitstellbaren Drehfeldleistung zu entladen oder aufzuladen.

7. Gantryantrieb (20) nach Anspruch 6,
   wobei der Energiepuffer (26) mit dem Drehstrommotor verschaltet und mittels der Regeleinheit (24) derart regelbar ist, dass zusätzlich zum ersten Anteil eine weitere mechanische Antriebsleistung zur Erzeugung der Drehbewegung des Drehstromrotors (22) mittels Energie aus dem Energiepuffer (26) bereitstellbar ist.

8. Gantryantrieb (20) nach einem der Ansprüche 6 oder 7,

wobei der Energiepuffer (26) mit dem Drehstrommotor verschaltet und mittels der Regeleinheit (24) derart regelbar ist, dass im Generatorbetrieb des Gantryantriebs (20) der Energiepuffer (26) aufladbar ist.

9. Gantryantrieb (20) nach einem der Ansprüche 6 bis 8,
wobei der Energiepuffer (26) mit der Nutzlasteinrichtung (25) derart verschaltet und mittels der Regeleinheit (24) derart regelbar ist, dass zusätzlich zum zweiten Anteil eine weitere elektrische Versorgungsleistung der Nutzlasteinrichtung (25) mittels Energie aus dem Energiepuffer (26) bereitstellbar ist.

10. Gantryantrieb (20) nach einem der Ansprüche 6 bis 9,
wobei der Energiepuffer (26) derart mit dem Drehstrommotor und der Nutzlasteinrichtung (25) verschaltet ist, dass die Drehfeldleistung ausschließlich mittels Energie aus dem Energiepuffer (26) bereitstellbar ist.

11. Computertomographieeinrichtung (30), aufweisend

- einen Gantryantrieb (20) nach einem der vorhergehenden Ansprüche,
- einen stationären Trägerring (31) und
- einen rotierenden Trägerring (32),
- wobei der stationäre Trägerring (31) mit dem Drehstromstator (21) verbunden ist und der rotierende Trägerring (32) drehfest relativ zum Drehstromrotor (21) angeordnet ist.

12. Computertomographieeinrichtung (30) nach Anspruch 11, ferner aufweisend einen Röntgendetektor (33),

- wobei mittels der Drehfeldleistung die elektrische Versorgungsleistung einem Röntgenstrahler der Nutzlasteinrichtung (25) bereitstellbar ist.

13. Verfahren zum Erzeugen einer Drehbewegung eines Drehstromrotors (22) mittels eines Gantryantriebs (20) nach einem der Ansprüche 1 bis 10, umfassend folgende Schritte:

- Bereitstellen der Drehfeldleistung in einem Drehstrommotor derart, dass einerseits ein erster Anteil der Drehfeldleistung als mechanische Antriebsleistung zur Erzeugung der Drehbewegung des Drehstromrotors des Drehstrommotors wirkt und dass andererseits ein zweiter Anteil der Drehfeldleistung als elektrische Versorgungsleistung der Nutzlasteinrichtung wirkt,
- Variieren des ersten Anteils und des zweiten Anteils.

14. Verfahren nach Anspruch 13,
wobei mittels einer Regeleinheit (24) der erste Anteil derart eingestellt wird, dass in einer Beschleunigungsphase des Drehstromrotors (22) der erste Anteil im Vergleich zum ersten Anteil nach der Beschleunigungsphase erhöht ist.

15. Verfahren nach einem der Ansprüche 13 bis 14,
wobei mittels einer Regeleinheit (24) der zweite Anteil derart eingestellt wird, dass nach einer Beschleunigungsphase des Drehstromrotors (22) der zweite Anteil im Vergleich zum zweiten Anteil in der Beschleunigungsphase erhöht ist.

16. Computerprogrammprodukt, welches direkt in einen Speicher einer Recheneinheit einer Regeleinheit eines Gantryantriebs (20) ladbar ist, mit Programmcodemitteln, um ein Verfahren nach einem der Ansprüche 13 bis 15 auszuführen, wenn das Computerprogrammprodukt in der Recheneinheit ausgeführt wird.

## Claims

1. Gantry drive (20) having:

- a multiphase stator (21);
- a multiphase rotor (22) and;
- a tunnel-shaped cavity (23) for receiving a patient,
- wherein the multiphase rotor (22) spatially surrounds the cavity (23),
- wherein the multiphase stator (21) and the multiphase rotor (22) form a multiphase motor for providing a rotating-field power,
- wherein by means of the rotating-field power, in the form of a first portion, can be provided a mechanical drive power for producing a rotational movement of the multiphase rotor (22), and, in the form of a second portion, can be provided an electrical supply power for a payload apparatus (25),

**characterised in that** the gantry drive comprises a control unit (24) which is designed to vary the first portion and the second portion.

2.  Gantry drive (20) according to claim 1, wherein the control unit (24) is designed to increase the first portion or the second portion at the expense of the second portion or the first portion respectively.

3.  Gantry drive (20) according to claim 2, wherein the control unit (24) is designed to increase the first portion at the expense of the second portion in an acceleration stage of the multiphase rotor (22), and/or to increase the second portion at the expense of the first portion after the acceleration stage of the multiphase rotor (22).

4.  Gantry drive (20) according to one of the preceding claims, wherein the control unit (24) is designed to drive the multiphase motor as a transformer to modulate the magnetic flux in order to vary the first portion and/or the second portion.

5.  Gantry drive (20) according to one of the preceding claims, wherein the control unit (24) is designed to vary the first portion and/or the second portion in accordance with field-oriented control.

6.  Gantry drive (20) according to one of the preceding claims,
    wherein the gantry drive (20) has an energy buffer (26), which is electrically connected to the multiphase motor, wherein the control unit (24) is designed to discharge or charge the energy buffer (26) according to the rotating-field power that can be provided.

7.  Gantry drive (20) according to claim 6,
    wherein the energy buffer (26) is connected to the multiphase motor, and can be controlled by means of the control unit (24) in such a way that in addition to the first portion, a further mechanical drive power for producing the rotational movement of the multiphase rotor (22) can be provided by means of energy from the energy buffer (26).

8.  Gantry drive (20) according to one of claims 6 or 7,
    wherein the energy buffer (26) is connected to the multiphase motor, and can be controlled by means of the control unit (24) in such a way that the energy buffer (26) can be charged when the gantry drive (20) is operating as a generator.

9.  Gantry drive (20) according to one of claims 6 to 8,
    wherein the energy buffer (26) is connected to the payload apparatus (25) in such a way, and can be controlled by means of the control unit (24) in such a way, that in addition to the second portion, a further electrical supply power for the payload apparatus (25) can be provided by means of energy from the energy buffer (26).

10. Gantry drive (20) according to one of claims 6 to 9,
    wherein the energy buffer (26) is connected to the multiphase motor and to the payload apparatus (25) in such a way that the rotating-field power can be provided solely by means of energy from the energy buffer (26).

11. Computed tomography apparatus (30) having:

    - a gantry drive (20) according to one of the preceding claims;
    - a stationary carrier ring (31); and
    - a rotating carrier ring (32),
    - wherein the stationary carrier ring (31) is joined to the multiphase stator (21), and the rotating carrier ring (32) is mounted for conjoint rotation with the multiphase rotor (21).

12. Computed tomography apparatus (30) according to claim 11, further comprising an X-ray detector (33)

    - wherein the electrical supply power can be provided to an X-ray source of the payload apparatus (25) by means of the rotating-field power.

13. Method for producing a rotational movement of a multiphase rotor (22) by means of a gantry drive (20) according to one of claims 1 to 10, comprising the following steps:

- providing the rotating-field power in a multiphase motor in such a way that a first portion of the rotating-field power acts as a mechanical drive power for producing the rotational movement of the multiphase rotor of the multiphase motor, and a second portion of the rotating-field power acts as the electrical supply power for the payload apparatus,
- varying the first portion and the second portion.

14. Method according to claim 13,
wherein by means of a control unit (24), the first portion is adjusted in such a way that in an acceleration stage of the multiphase rotor (22), the first portion is higher compared with the first portion after the acceleration stage.

15. Method according to one of claims 13 to 14,
wherein by means of a control unit (24), the second portion is adjusted in such a way that after an acceleration stage of the multiphase rotor (22), the second portion is higher compared with the second portion in the acceleration stage.

16. Computer program product, which can be loaded directly into a memory of a processing unit of a control unit of a gantry drive (20) and has program code means in order to perform a method according to one of claims 13 to 15 when the computer program product is executed in the processing unit.

**Revendications**

1. Entraînement (20) de portique, comportant

- un stator (21) à courant triphasé,
- un rotor (22) à courant triphasé et
- une cavité (23) en forme de tunnel pour la réception d'un patient,
- dans lequel le rotor (22) à courant triphasé entoure dans l'espace la cavité (23),
- dans lequel le stator (21) à courant triphasé et le rotor (22) à courant triphasé forment un moteur à courant triphasé, pour disposer d'une puissance à champ tournant,
- dans lequel, au moyen de la puissance à champ tournant, peuvent être mises à disposition d'une part, sous la forme d'une première proportion, une puissance d'entraînement mécanique, pour la production d'un mouvement de rotation du rotor (22) à courant triphasé, et d'autre part, sous la forme d'une deuxième proportion, une puissance d'alimentation électrique d'un dispositif (25) de charge utile,

**caractérisé en ce que** l'entraînement de portique comprend une unité (24) de réglage, qui est constituée pour faire varier la première proportion et la deuxième proportion.

2. Entraînement (20) de portique suivant la revendication 1, dans lequel l'unité (24) de réglage est constituée pour augmenter la première proportion ou la deuxième proportion au détriment de la deuxième proportion ou de la première proportion.

3. Entraînement (20) de portique suivant la revendication 2, dans lequel l'unité (24) de réglage est constituée pour augmenter la première proportion au détriment de la deuxième proportion dans une phase d'accélération du rotor (22) à courant triphasé et/ou pour augmenter la deuxième proportion au détriment de la première proportion, après la phase d'accélération du rotor (22) à courant triphasé.

4. Entraînement (20) de portique suivant l'une des revendications précédentes,
dans lequel l'unité (24) de réglage est constituée pour commander le moteur à courant triphasé comme transformateur de modulation du flux magnétique, afin de faire varier la première proportion et/ou la deuxième proportion.

5. Entraînement (20) de portique suivant l'une des revendications précédentes,
dans lequel l'unité (24) de réglage est constituée pour faire varier la première proportion et/ou la deuxième proportion suivant un réglage en fonction du champ.

6. Entraînement (20) de portique suivant l'une des revendications précédentes,
dans lequel l'entraînement (20) de portique a un tampon (26) d'énergie connecté électriquement au moteur à courant triphasé, dans lequel l'unité (24) de réglage est constituée pour décharger ou pour charger le tampon (26) d'énergie en fonction de la puissance à champ tournant pouvant être mise à disposition.

**7.** Entraînement (20) de portique suivant la revendication 6, dans lequel le tampon (26) d'énergie connecté au moteur à courant continu peut être réglé au moyen de l'unité (24) de réglage, de manière à mettre à disposition, au moyen de l'énergie du tampon (26) d'énergie, en plus de la première proportion, une autre puissance d'entraînement mécanique pour la production du mouvement de rotation du rotor (22) à courant triphasé.

**8.** Entraînement (20) de portique suivant l'une des revendications 6 ou 7,
dans lequel le tampon (26) d'énergie connecté au moteur à courant triphasé peut être réglé au moyen de l'unité (24) de réglage, de manière à pouvoir charger le tampon (26) d'énergie en fonctionnement en générateur de l'entraînement (20) de portique.

**9.** Entraînement (20) de portique suivant l'une des revendications 6 à 8,
dans lequel le tampon (26) d'énergie connecté au dispositif (25) de charge utile peut être réglé au moyen de l'unité (24) de réglage, de manière à mettre, au moyen de l'énergie du tampon (26) d'énergie supplémentairement à la deuxième proportion, une autre puissance d'alimentation électrique du dispositif (25) de charge utile.

**10.** Entraînement (20) de portique suivant l'une des revendications 6 à 9,
dans lequel le tampon (26) d'énergie est connecté au moteur à courant triphasé et au dispositif (25) de charge utile, de manière à pouvoir mettre à disposition la puissance à champ tournant exclusivement au moyen de l'énergie du tampon (26) d'énergie.

**11.** Dispositif (30) de tomodensitométrie assistée par ordinateur, comportant

- un entraînement (20) de portique suivant l'une des revendications précédentes,
- un anneau (31) porteur fixe et
- un anneau (32) porteur tournant,
- dans lequel l'anneau (31) porteur fixe est assemblé au stator (21) à courant triphasé et l'anneau (32) porteur tournant est fixe en rotation relativement au rotor (21) à courant triphasé.

**12.** Dispositif (30) de tomodensitométrie assistée par ordinateur suivant la revendication 11, comportant en outre un détecteur (33) de rayons X,

- dans lequel, au moyen de la puissance à champ tournant, la puissance d'alimentation électrique peut être donnée à un émetteur de rayons X du dispositif (25) de charge utile.

**13.** Procédé de production d'un mouvement de rotation d'un rotor (22) à courant triphasé au moyen d'un entraînement (20) de portique suivant l'une des revendications 1 à 10, comprenant les stades suivants :

- donner la puissance à champ tournant à un moteur à courant triphasé, de manière à ce que d'une part une première proportion de la puissance à champ tournant agisse comme puissance d'entraînement mécanique, pour la production du mouvement de rotation du rotor à courant triphasé du moteur à courant triphasé, et de manière à ce que d'autre part une deuxième proportion de la puissance à champ tournant agisse comme puissance d'alimentation électrique du dispositif de charge utile,
- faire varier la première proportion et la deuxième proportion.

**14.** Procédé suivant la revendication 13,
dans lequel, au moyen d'une unité (24) de réglage, on règle la première proportion, de manière à augmenter, dans une phase d'accélération du moteur (22) à courant triphasé, la première proportion par rapport à la première proportion, après la phase d'accélération.

**15.** Procédé suivant l'une des revendications 13 à 14,
dans lequel, au moyen d'une unité (24) de réglage, on règle la deuxième proportion, de manière à augmenter, après une phase d'accélération du rotor (22) à courant triphasé, la deuxième proportion par rapport à la deuxième proportion dans la phase d'accélération.

**16.** Produit de programme d'ordinateur, qui peut être chargé directement dans la mémoire d'une unité informatique d'une unité de réglage d'un entraînement (20) de portique, comprenant des moyens de code de programme, pour exécuter le procédé suivant l'une des revendications 13 à 15, lorsque le produit de programme d'ordinateur est exécuté dans l'unité informatique.

## FIG 1
(Stand der Technik)

## FIG 2

# FIG 3

# FIG 4

EP 4 285 824 B1

**FIG 5**

**FIG 6**

21

EP 4 285 824 B1
EP 4 285 824 B1

## FIG 7

## FIG 8

FIG 9

**FIG 10**

**FIG 11**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0181176 A2
- US 2002031201 A1
- JP 2004202092 A
- DE 102014201805 A1
- US 2017214279 A1